Europäisches Patentamt

European Patent Office

Office européen des brevets

(11) Veröffentlichungsnummer: **0 410 179 A2**

## (12) EUROPÄISCHE PATENTANMELDUNG

(21) Anmeldenummer: **90112888.4**

(22) Anmeldetag: **06.07.90**

(51) Int. Cl.5: **A61B 17/36**

(30) Priorität: **25.07.89 DE 3924491**

(43) Veröffentlichungstag der Anmeldung:
**30.01.91 Patentblatt 91/05**

(84) Benannte Vertragsstaaten:
**BE DE FR GB IT**

(71) Anmelder: **Richard Wolf GmbH**
**Pforzheimer Strasse 32**
**D-7134 Knittlingen(DE)**

(72) Erfinder: **Steiner, Rudolf, Prof. Dr.**
**Maienweg 69**
**D-7900 Ulm/Söflingen(DE)**
Erfinder: **Xiao-Meng, Liu, Dr. c/o Prof.Dr.Rud.**
**Steiner in**
**Universität Ulm, Helmholtzstr. 12, Postfach**
**4066**
**D-7900 Ulm(DE)**

(74) Vertreter: **Wilcken, Thomas, Dipl.-Ing. et al**
**Musterbahn 1**
**D-2400 Lübeck(DE)**

(54) **Laserlichtapplikator.**

(57) Der Laserlichtapplikator besteht aus einer Laser-Einzellichtleitfaser (1), die distal stumpf mit einem zylindrischen Lichtleiterteil (2) verbunden ist, welcher zur Stabilisierung der Verbindung von einer Hülse ummantelt ist. Der Lichtleiterteil (2) ist distal als Kugelkopf (3) mit planen, einen Keil zwischen sich bildenden, geschwärzten, mit stumpfer Keilkante (4) auslaufenden Anschliff-Flächen (5) versehen. Der Kugelkopf (3) besitzt einen Durchmesser von 1,5 bis 2,0 mm und der Keilwinkel beträgt zwischen 54 bis 60°.

FIG. 4

EP 0 410 179 A2

## LASERLICHTAPPLIKATOR

Die Erfindung geht von einem Laserlichtapplikator nach der EP-PS 0 125 897 aus, bei dem eine Laserlichtleitfaser distal mit einem im Durchmesser vergrößerten, keilförmig auslaufenden, mit der Laserfaser verschweißten Lichtleitteil als Schneidmesser verbunden ist.

Es hat sich gezeigt, daß der Keil an sich eine gute Führung beim Verdampfen von Gewebe darstellt, daß aber die spitz auslaufende Keilkante durch im Keil auftretende Strahlungsenergie sehr stark erhitzt und dadurch zerstört wird.

Die Aufgabe der Erfindung besteht darin, bei verhältnismäßig geringem Energieaufwand eine hohe Hitzeentwicklung mit dem Keil zu erzeugen, dabei aber eine Zerstörung der Keilkante zu vermeiden.

Diese Aufgabe wird durch die Merkmale des Anspruches 1 gelöst.

Durch die Schwärzung der Keilflächen wird eine hohe Absorption des Laserlichtes erzeugt, die eine Keilspitze zu stark aufheizen würde, was nach der Erfindung dadurch vermieden wird, daß der Keilsteg stumpf ausgebildet ist.

Als weitere Ausbildung der Erfindung wird vorgesehen, daß die Keilflächen, insbesondere bei einem Durchmesser des Kugelkopfes zwischen 1,5 bis 2,0 mm, einen Winkel von 54 bis 60° besitzen. Bei größeren Durchmessern werden die Flanken bzw. Keilflächen nicht mehr heiß genug und bei größeren Keilwinkeln besteht die Gefahr, daß die Strahlung von den geschwärzten Keilflächen zurückreflektiert wird und auf die vorzusehende Ummantelung der Laserfaser trifft, diese aufheizt und zerstört. Bei kleineren Winkeln könnte die Strahlung auf den Keilsteg reflektiert werden, wodurch dieser stark erhitzt wird und leichter zerstört werden kann.

Die Erfindung ist nachstehend anhand der Zeichnung erläutert. Es zeigen:

Figur 1 die Seitenansicht des distalen Endes einer Laserlichtleitfaser mit der Ausbildung nach der Erfindung,

Figuren 2 bis 4 drei Phasen zur Herstellung des distalen Endes der Lichtleitfaser in Seitenansicht.

Der Laserlichtleitapplikator ist so ausgebildet, daß eine Laserlichtleitfaser 1 distal stumpf mit einem zylindrischen Lichtleiter 2 verschweißt bzw. verschmolzen ist, der in einen Kugelkopf 3 mit einem Durchmesser zwischen 1,5 bis 2,0 mm übergeht, der mit zwei planen, einen Keil mit stumpfer Keilkante 4 bildenden Anschliff-Flächen 5 versehen ist, die insbesondere einen Winkel zwischen 54 bis 60° miteinander bilden. Die Übergangsstelle zwischen Lichtleitfaser 1 und dem zylindrischen Lichtleiter 2 ist zur Stabilisierung von einer Zylinderhülse 6 umgeben.

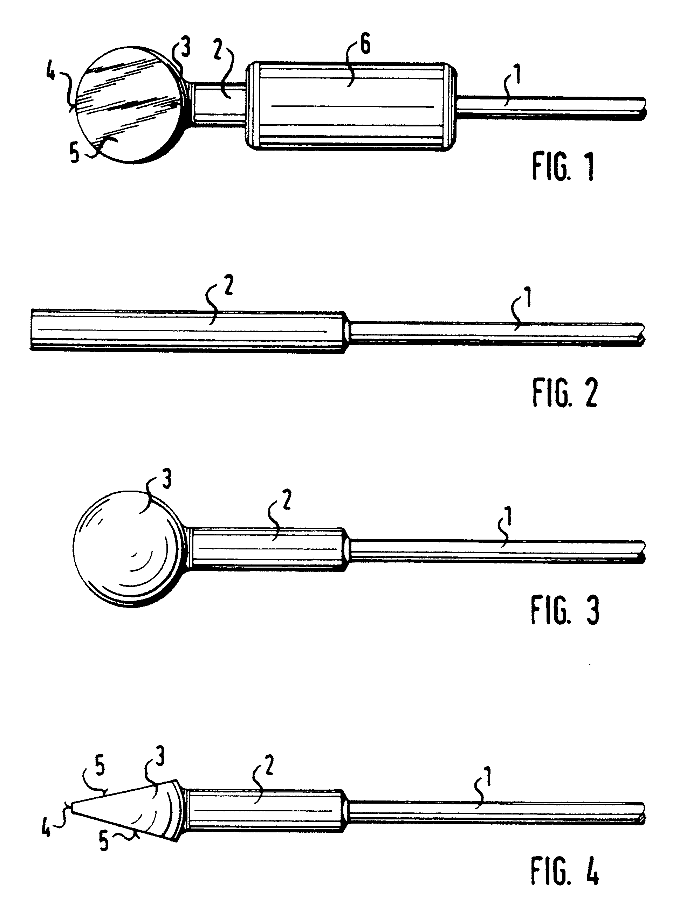

Bei der Herstellung des Laserlichtapplikators wird so vorgegangen, daß nach der Verbindung der Einzelfaser 1 mit dem Lichtleiterteil 2 (Figur 2) der letztere distal durch Wärmezufuhr in einen Kugelkopf 3 umgeformt wird (Figur 3). Es wird sodann der Kugelkopf 3 mit zwei planen, einen Keil bildenden Anschliff-Flächen 5 versehen, wobei die beiden Anschliff-Flächen an der Keilspitze eine stumpfe Kante 4 bilden. Schließlich wird eine Ummantelung im Bereich des Überganges zwischen der Einzelfaser 1 und dem Lichtleiterteil 2 in Form der Zylinderhülse 6 zur Stabilisierung angebracht.

## Ansprüche

1. Laserlichtapplikator, bei dem eine Laserlichtleitfaser distal mit einem im Durchmesser vergrößerten, keilförmig auslaufenden Lichtleiterteil als Schneidmesser verbunden ist, dadurch gekennzeichnet, daß der im Durchmesser gegenüber der Laserfaser (1) vergrößerte Lichtleiterteil (2) in einem kugelartigen Kopf (3) endet, der durch zwei plane, geschwärzte Anschliff-Flächen (5) als Keil mit abgestumpften, nicht geschschwärztem Keilsteg (4) ausgebildet ist.

2. Laserlichtapplikator nach Anspruch 1, dadurch gekennzeichnet, daß die beiden Anschliff-Flächen (5) des einen Durchmesser von 1,5 bis 2,0 mm aufweisenden Kugelkopfes (3) einen Winkel von 54 - 60° einschließen.

3. Laserlichtapplikator nach Anspruch 1 oder 2, dadurch gekennzeichnet, daß der abgestumpfte Keilsteg (4) bogenförmig in die verbleibende Kugelfläche des Kugelkopfes (3) übergeht.

FIG. 1

FIG. 2

FIG. 3

FIG. 4